# EUROPEAN PATENT SPECIFICATION

(11) **EP 0 532 577 B1**
(45) Date of publication and mention of the grant of the patent: **11.01.1995**
(21) Application number: 91910398.6
(22) Date of filing: 31.05.1991
(51) Int. Cl.: C07C 43/17, C07C 43/174, C07C 43/12, C07C 43/13, C07C 69/653, C07C 205/37, C07C 217/84, C07D 303/20, C08F 14/18

(54) **FUNCTIONALIZED TRIFLUOROVINYL ETHERS AND POLYMERS THEREFROM**
FUNKTIONALISIERTE TRIFLUORVINYLETHER UND POLYMERE DAVON
ETHERS TRIFLUOROVINYLIQUES FONCTIONNALISES ET POLYMERES OBTENUS A PARTIR DE CES ETHERS

(30) Priority: 04.06.1990 US 532618
(43) Date of publication of application: 24.03.1993
(73) Proprietor: E.I. DU PONT DE NEMOURS AND COMPANY, Wilmington Delaware 19898 (US)
(72) Inventor: HUNG, Ming-Hong, Wilmington, DE 19810 (US)
(74) Representative: Freiherr von Wittgenstein, Arved, Dr.
(86) International application number: US9103649
(87) International publication number: WO9118859

(56) References cited:
- EP-A- 0 135 917
- EP-A- 0 199 138
- US-A- 4 564 717
- PATENT ABSTRACTS OF JAPAN, vol. 7, no. 181 (C-180)[1326], 10 August 1983; & JP-A-58 85 832

## Description

This invention concerns selected trifluorovinyl ethers containing various functional groups, and polymers prepared from them by free radical polymerization. Also provided are intermediates for use in the preparation of the trifluorovinyl ethers.

### BACKGROUND OF THE INVENTION

Japanese Patent 88002418 reports the synthesis of 7,7-dihydro-7-hydroxy(perfluoro-3-oxahepten-1) by chlorinating the methyl ester of perfluoro(3-oxa-1-heptenoic acid), reduction of the chlorinated product with NaBH₄ to give the corresponding alcohol, and then reaction of the alcohol with zinc metal to regenerate the vinyl ether, which is the desired product. It is reported that this compound can be free radically copolymerized with at least one other fluorinated monomer, and optionally non-fluorinated monomers to form useful polymers.

U. S. Patent 4,564,717 reports the synthesis of compounds of the formula CF₂=CF(CF₂)ₘ(CH₂)ₙOH wherein m is an integer from 0 to 10 and n is an integer of 1 to 4. Among the methods of preparation described, is the reduction of the compound CF₂X¹CFX²CF₂COOR (sic) wherein R is alkyl and X¹ and X² are chlorine or bromine, by various reducing agents. The olefin is then produced by dehalogenation of the alcohol with a metal such as zinc.

European Patent Application 135,917 discloses copolymers of vinylidene fluoride with a compound of the formula CF₂=CF(CF₂)ₘ(CH₂)ₙOH wherein m is 0 to 10 and n is 1-4, and optionally another fluorinated termonomer. Polymers containing fluorovinyl ethers are not disclosed.

European Patent Application 199,138 reports preparation and polymerization (with other fluorine containing olefins) of the compound CF₂=CFO(CF₂CFYO)ₙ(CF₂CF₂CH₂O)ₘCF₂CF₂CH₂X, wherein X is hydrogen or halogen, Y is fluorine or -CF₃, m is an integer of 0 to 5 and n is 0, 1 or 2. No mention is made of other functional groups being present.

It is the object of this invention to provide trifluorovinyl ether monomers that contain various functional groups. The functional groups may be chemically relatively inert, such as alkyl ether, which when used in a minor proportion in a fluorinated polymer, changes the surfaces properties of that polymer. The functional group may also be more chemically active, such as epoxy, which may be utilized as a crosslinking or adhesion promotion site. Finally the functional group may be styryl or acrylic so that the functional group may be readily polymerized. Also provided are the homo- and copolymers of such compounds, and intermediate compounds from which such trifluorovinyl ethers are made.

### SUMMARY OF THE INVENTION

This invention comprises a trifluorovinyl ether of the formula I

CF₂=CF-[OCF₂CF(CF₃)]ₚ-[O(CF₂)_{q}]ᵣ-CH₂OR¹ I

wherein:
R¹ is alkyl or cycloalkyl containing 1 to 30 carbon atoms, 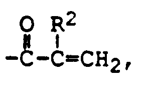
-(CH₂)ₛR³ or -(CH₂)ₜR⁵;
R² is hydrogen or methyl;
R³ is -C≡CH, -CH=CH₂, styryl or 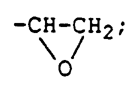
R⁵ is aryl or substituted aryl;
p is an integer of 0 to 20;
q is an integer of 1 to 10;
r is 0 or 1;
s is 1, 2, 3 or 4; and
t is 0, 1, 2, 3 or 4;
provided that when r is 0, p is not 0.

A further aspect of this invention comprises halogenated compounds of the formula II

CF₂XCFX-[OCF₂CF(CF₃)]ₚ-[O(CF₂)_{q}]ᵣ-CH₂OR¹ II

wherein:
R¹ is alkyl or cycloalkyl containing 1 to 30 carbon atoms, 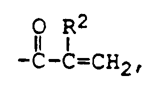
-(CH₂)ₛR³, -(CH₂)ₜR⁵ or hydrogen;
R² is hydrogen or methyl;
R³ is -C≡CH, -CH=CH₂, styryl or 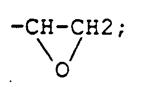
R⁵ is aryl, or substituted aryl;
each X is independently chlorine or bromine;
p is an integer of 0 to 20;
q is an integer of 1 to 10;
r is 0 or 1;
s is 1, 2, 3 or 4; and
t is 0, 1, 2, 3, or 4;
provided that when r is 0, p is not 0, and further provided that when p is 0, q is three, r is 1, and R¹ is not hydrogen.

A further aspect of this invention comprises copolymers of trifluorovinyl ethers comprising units of the formula III 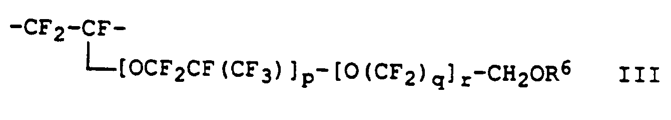
and one or more other free radically polymerizable monomer wherein:
R⁶ is alkyl or cycloalkyl containing 1 to 30 carbon atoms, -(CH₂)ₛR⁸, or -(CH₂)ₜR⁵;
R⁸ is -C≡CH, -CH=CH₂, or 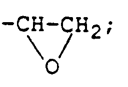
R⁵ is aryl, and substituted aryl;
p is an integer of 0 to 20;
q is an integer of 1 to 10;
r is 0 or 1;
s is 1, 2, 3 or 4; and
t is 0, 1, 2, 3, or 4;
provided that when r is 0, p is not 0.

Also provided is a polymer made by the free radical polymerization of a compound of the formula IV

CF₂=CF-[OCF₂CF(CF₃)]ₚ-[O(CF₂)_{q}]ᵣ-CH₂OR⁷ IV

and optionally other vinyl monomers, wherein:
R⁷ is 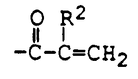
or -(CH₂)ₛR⁴;
R² is hydrogen or methyl;
R⁴ is styryl;
p is an integer of 0 to 20;
q is an integer of 1 to 10;
r is 0 or 1; and
s is 1, 2, 3 or 4;
provided that when r is 0, p is not 0.

### DETAILS OF THE INVENTION

The term "styryl" is used herein to mean the group o-, m-, or p-vinylphenyl, which may have inert substituents bound to the benzene ring.

By the term "substituted aryl" herein is meant an aryl group substituted with any group that does not interfere with, and is stable during, any of the reactions the particular compound containing that group has or will undergo. Examples of suitable substituents include, but are not limited to, halo, ester, alkyl, ether, aryl, and cycloalkyl.

The trifluorovinyl ethers of this invention may be prepared from the halogenated compounds of this invention, which in turn are made by known processes from known starting materials. Thus the halogenated compound of formula II wherein both Xs are chlorine, p is 1, q is 2, r is 1 and R¹ is hydrogen is made by the chlorination of CF₂=CFOCF₂CF(CF₃)OCF₂CF₂COOCH₃. See U.S. Patent 4,138,426, which is herein incorporated by reference, for preparation of this compound, and subsequent reduction of the methyl ester to the alcohol (see Example 1). This alcohol, and others such as those described in Japanese Patent 88002418, can then be converted to ethers or esters using the reactions described herein. That is, R¹ is changed from hydrogen to one of the other groups listed for R¹ supra, in the formula of the halogenated compound.

Ethers may be made by variations of the Williamson ether synthesis, a reaction well known to those skilled in the art. Briefly, an alcohol is converted to its alkoxide, and the alkoxide is reacted with a (cyclo)alkyl halide to yield the corresponding ether. The alkyl halide may be substituted with various functional groups, so long as these group do not react with the alkoxide faster than the halide does. Such reactions are illustrated in Examples 2-7 herein. A general reference on such reactions is H. Feuer and J. Hooz in S. Patai, Ed., The Chemistry of the Ether Linkage, Interscience Publishers, London, 1967, p. 446-448.

The halogenated compounds in which R¹ is hydrogen (that is halogenated compound "alcohols") may be converted to their corresponding acrylic esters by standard esterification reactions. For example the alcohol may be reacted with an acrylyl or methacrylyl halide, preferably the chloride, to form the (meth)acrylic ester. Such reactions, and others useful for esterification, are well known to those skilled in the art.

In preferred embodiments of the halogenated compounds of formula II, p is 0, r is 1 and q is 1 to 10; or p is 1, r is 1 and q is 2. In another preferred embodiment of the halogenated compound both groups X are chlorine. Preferred groups R¹ are: n-alkyl; -(CH₂)ₛR³ wherein s is 1 and R³ is -C≡CH, -CH=CH₂ or styryl; or -(CH₂)ₜR⁵ wherein t is 0 or 1. Especially preferred R¹ groups are (CH₂)ₛR³ wherein s is 1 and R³ is -CH=CH₂ or styryl. It is also preferred that when t is 0, the group R⁵ be substituted with, or have substituents derived from, electron withdrawing groups, such as nitro or cyano. Amino, for example, is a substituent derived from an electron withdrawing group, namely nitro. Also preferred are any and all combinations of the above preferred embodiments.

Preferred specific halogenated compounds are:
3,6,10-trioxa-1,2-dichloro-1,1,2,4,4,5,7,7,8,8-decafluoro-5-trifluoromethyl-12,13-epoxytridecane;
3,6,10-trioxa-1,2-dichloro-1,1,2,4,4,5,7,7,8,8-decafluoro-5-trifluoromethylhexadedane;
3,6,10-trioxa-1,2-dichloro-1,1,2,4,4,5,7,7,8,8-decafluoro-5-trifluoromethyloctacosane;
4,8,11-trioxa-12,13-dichloro-6,6,7,7,9,10,10,12,13,13-decafluoro-9-trifluoromethyltridec-1-yne;
3,6,10-trioxa-1,2-dichloro-1,1,2,4,4,5,7,7,8,8-decafluoro-5-trifluoromethyl-11-(vinylphenyl)undecane;
(3,6-dioxa-1,2-dichloro-1,1,2,4,4,5,7,7,8,8-decafluoro-5-trifluoromethylnonyl) 2,4-dinitrophenyl ether; and
(3,6-dioxa-1,2-dichloro-1,1,2,4,4,5,7,7,8,8-decafluoro-5-trifluoromethylnonyl) 2,4-diaminophenyl ether.

The trifluorovinyl ethers of formula I of the present invention may be prepared by the dehalogenation of the above described halogenated compounds, by direct reaction of the corresponding trifluorovinyl ether alcohol, or by reaction of the group R¹ of a trifluorovinyl ether to form a different R¹ group. The latter two methods are preferred if the final group on the trifluorovinyl ether is relatively reactive, especially in a dehalogenation reaction.

The dehalogenation of 1,2-dichloro- or 1,2-dibromotrifluoroethoxy groups with an active metal such as zinc to form trifluorovinyl ether groups is known to those skilled in the art, for example see Japanese Patent 88002418 and U. S. Patent 4,564,717, supra. This reaction is illustrated in Examples 9-12 herein. The groups R¹ should be stable to the reaction conditions.

The reaction of the corresponding trifluorovinyl ether alcohol to form a trifluorovinyl ether claimed herein is particularly useful where a functional group that may be reactive in the dehalogenation reaction is desired. Such a reaction is the esterification of the alcohol to form an acrylic ester. For example, the alcohol may be reacted with a (meth)acrylyl halide, preferably the chloride, to form a (meth)acrylic ester. This and similar esterification reactions are known to those skilled in the art. Example 13 herein illustrates such a reaction.

Finally certain of the trifluorovinyl ethers claimed herein may be made by reaction of a functional group within R¹ to form a different functional group. This reaction is also particularly useful when the final functional group may not be stable to the dehalogenation reaction. An example of such a reaction is the epoxidation of a vinyl group to an epoxide. For example, when R¹ is -(CH₂)ₛR³, and R³ is -CH=CH₂, the vinyl group may be oxidized by typical epoxidation reactions (for example see Kirk-Othmer Encyclopedia of Chemical Technology, 3rd Ed., vol. 9, John Wiley & Sons, New York, 1980, p. 251-266), such as oxidation with m-chloroperbenzoic acid. Such an oxidation may also be performed on a styryl group.

In preferred embodiments of the fluorovinyl ethers of Formula I, p is 0, r is 1 and q is 1 to 10; or p is 1, r is 1 and q is 2. Preferred groups R¹ are: n-alkyl; -(CH₂)ₛR³ wherein s is 1 and R³ is -C≡CH, -CH=CH₂, styryl, or 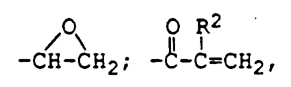
wherein R² is hydrogen or methyl; or -(CH₂)ₜR⁵ wherein t is 0 or 1. Especially preferred R¹ groups are -(CH₂)ₛR³, wherein s is 1 and R³ is -C=CH₂, styryl, or 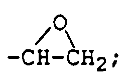
or 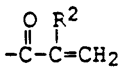
wherein R² is hydrogen or methyl. It is also preferred that when t is 0, the group R⁵ be substituted with, or have substituents derived from, electron withdrawing groups, such as nitro or cyano. Amino, for example, is a substituent derived from an electron withdrawing group, namely nitro. Also preferred are any and all combinations of the above preferred embodiments.

Preferred specific trifluorovinyl ethers are:
3,6,10-trioxa-5-trifluoromethyl-1,1,2,4,4,5,7,7,8,8-decafluorohexadec-1-ene;
3,6,10-trioxa-5-trifluoromethyl-1,1,2,4,4,5,7,7,8,8-decafluorooctacos-1-ene;
4,8,11-trioxa-9-trifluoromethyl-6,6,7,7,9,10,10,12,13,13-decafluorotridec-12-ene-1-yne;
3,6,10-trioxa-5-trifluoromethyl-1,1,2,4,4,5,7,7,8,8-decafluoro-11-(vinylphenyl)undec-1-ene; and
4,7-dioxa-5-trifluoromethyl-2,2,3,3,5,6,6,8,9,9-decafluoronon-8-enyl methacrylate.

The copolymer of trifluorovinyl ethers comprising units of the formula III 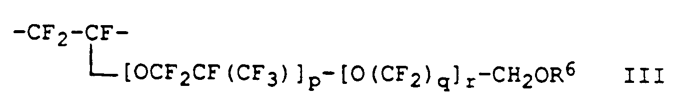
is made by the free radical copolymerization of the trifluorovinyl ethers described above with one or more other free radically polymerizable monomer. Free radical polymerization is a process well known to those skilled in the art, and may be carried out in solution, or for example, in water emulsion. If the latter is used, any functional groups in the trifluorovinyl ether should be stable to water. Initiators that are typically used for the free radical polymerization of fluoroolefins may be used, for example benzoyl peroxide, 4,4'-bis(t-butyl-cyclohexyl)peroxy dicarbonate, di-t-butyl peroxide, and a combination of ferrous ion and persulfate ion.

Copolymers with ethylene or vinyl monomers are preferred. Especially preferred comonomers are fluorinated vinyl monomers. More preferred monomers are vinylidene fluoride, tetrafluoroethylene, vinyl fluoride, perfluoro(methyl vinyl ether), chlorotrifluoroethylene, perfluoro-1-heptene, hexafluoropropylene and perfluoro-2,2-dimethyl-1,3-dioxole. The most preferred comonomer is tetra-fluoroethylene. In a copolymer containing a fluorinated vinyl monomer, it is preferred if the trifluorovinyl ether is about 20 mole percent or less of the total amount of monomer units in the polymer. It is believed that even this relatively small amount of the fluorinated vinyl ether will supply enough reactive groups (for example, epoxy) to crosslink the polymer, if desired, or, if R¹ is for example alkyl, change the surface properties of the polymer. Such surface properties include wettability and the ability to adhere various items to the polymer surface.

In preferred trifluorovinyl ether polymers, p is 0, r is 1 and q is 1 to 10; or p is 1, r is 1 and q is 2. Preferred groups R⁶ are: n-alkyl; -(CH₂)ₛR³ wherein s is 1 and R³ is -C≡CH, -CH=CH₂, or 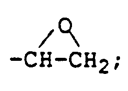
or -(CH₂)ₜR⁵ wherein t is 0 or 1. It is also preferred that when t is 0, the group R⁵ be substituted with, or have substituents derived from, electron withdrawing groups, such as nitro or cyano. Also preferred are any and all combinations of the above preferred embodiments.

This invention includes homo- or copolymers made by the free radical polymerization of a compound of the formula IV

CF₂=CF-[OCF₂CF(CF₃)]ₚ-[O(CF₂)_{q}]ᵣ-CH₂OR⁷ IV

and optionally ethylene or vinyl monomers, wherein:
R⁷ is 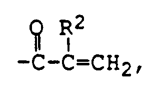
or -(CH₂)ₛR⁴;
R² is hydrogen or methyl;
R⁴ is styryl;
p is an integer of 0 to 20;
q is an integer of 1 to 10;
r is 0 or 1; and
s is 1, 2, 3 or 4;
provided that when r is 0, p is not 0. Such compounds are made by polymerization processes known to those skilled in the art. Thus the polymerization of these monomers is initiated by typical free radical initiators such as benzoyl peroxide, 4,4'-bis(t-butylcyclohexyl)peroxy dicarbonate, di-t-butyl peroxide, azobis(isobutyronitrile), or a combination of ferrous ion and persulfate ion. The polymerization may be carried out in solution, or may be done in emulsion.

Copolymers are formed with typical vinyl monomers, such as acrylic esters, acrylamides, styrene, vinyl acetate, propylene, tetrafluoroethylene, chlorotrifluoroethylene, vinyl fluoride, vinylidene fluoride, hexafluoropropylene, perfluoro-2,2-dimethyl-1,3-dioxole, and the like. Since the monomers of these polymers contain both an acrylic, styryl, acetylenic or olefinic functionality, as well as a trifluorovinyl ether, both of which may polymerize in free radical polymerizations, crosslinked polymers may result. It is believed, but Applicant does not wish to be bound, that the styryl or acrylic function is particularly easily polymerized. Again it is preferred if the trifluorovinyl ether containing monomer is about 20 mole percent or less of the total number of monomer units in the copolymer. This is sufficient for modification of the polymer surface properties and/or crosslinking of the polymer. Crosslinked polymers generally have better chemical resistance and retain their shape better at higher temperatures than uncrosslinked polymers. In this case, the polymer surface will be made hydrophobic by the presence of the trifluorovinyl ether group.

Preferred polymers are made from monomers where p is 0, r is 1 and q is 1 to 10; or p is 1, r is 1 and q is 2. In other preferred polymers, s is 1. Also preferred are any and all combinations of the above preferred embodiments.

The following abbreviations are used in the Examples:
Compound A - 4,7-dioxa-8,9-dichloro-5-trifluoromethyl-2,2,3,3,5,6,6,8,9,9-decafluoronona-1-ol
DMF - N,N-dimethylformamide
NMR - nuclear magnetic resonance (spectroscopy)
TFE - tetrafluoroethylene
THF - tetrahydrofuran.

### EXAMPLE 1

### Preparation of Compound A

Methyl 4,7-dioxa-5-trifluoromethyl-2,2,3,3,5,6,6,8,9,9,-decafluoronon-8-enoate (600 g, 1.422 mole) was chlorinated with neat chlorine gas by bubbling the Cl₂ gas into it at 0-5°C. The reaction was stopped when the conversion was almost complete. The resulting material was distilled to give the desired product as a clear, colorless liquid, yield 626 g (89.3%). Bp. 107°C/45 mm. H¹NMR (CDCl₃) : δ3.96 (s).

The compound prepared above (98.6 g, 0.2 mole) was dissolved in absolute ethanol (60 ml) at 0°C. In a separate flask sodium borohydride (4.6 g, 0.12 mole) was mixed with absolute ethanol (80 ml) also at 0°C. The NaBH₄/EtOH solution was then added slowly into the other solution while being kept at < 5°C. After the addition, the mixture was stirred at ambient temperature for 15 min, and then dumped into a mixture of 6N HCl (200 ml) and ice water (200 ml). The bottom layer was separated and the aqueous layer was extracted with ether. The organic layers were combined, washed with water repeatedly, and dried over magnesium sulfate. After the solvent was removed in vacuo, the residue was distilled to afford the desired compound as a clear, colorless liquid, yield 63 g (68%). Bp. 92-93°C/25 mm. H¹NMR (CDCl₃): δ4.04 (t, J = 14.1 Hz, 2H), 2.26 (s, br, 1H); F¹⁹ NMR (CDCl₃): -71.3 (m, 2F), -77.4 (m, 1F), -80.3 (q, J = 8.7 Hz, 3F), -82.4 to -86.0 (m, 4F), -126.7 (t, J = 13.8 Hz, 2F), -146.2 (m, 1F).

### EXAMPLE 2

### Preparation of 3,6,10-Trioxa-1,2-dichloro-1,1,2,4,4,5,7,7,8,8-decafluoro-5-trifluoromethyl-12,13-epoxytridecane

NaH (2 g as 60% oil suspension, 0.05 mole) was suspended in THF solvent (25 ml) at 0°C. Compound A (23.3 g, 0.05 mole) in THF (5 ml) was added slowly and the pot was controlled at < 10°C. After the addition, epibromohydrin (7 g, 0.051 mole) was introduced. Then the reaction was stirred at 40°C for overnight, and the product was dumped into a mixture of ice-water (200 ml) and 6N HCl (30 ml). Ether was added to extract the organic product. The organic layer was washed with water, dried over MgSO₄, ether was removed in vacuo, and the residue was distilled to give the desired product as a clear liquid, yield 12 g (46%). Bp. 85°C/3.0 mm. H¹NMR (CDCl₃): δ3.98 (m, 3H), 3.52 (dd, J = 12 Hz, 6 Hz, 1H), 3.17 (m, 1H), 2.83 (t, J = 9 Hz, 1H), 2.62 (m, 1H); F¹⁹NMR (CDCl₃) : -71.2 (m, 2F), -77.4 (m, 1F), -80.2 (q, J = 8.7 Hz, 3F), -82.4 to -85.9 (m, 4F), -123.8 (t, br, J = 13.3 Hz, 2F), -146.2 (q, br, J = 20.1 Hz, 1F).

### EXAMPLE 3

### Preparation of 3,6,10-Trioxa-1,2-dichloro-1,1,2,4,4,5,7,7,8,8-decafluoro-5-trifluoromethylhexadecane

Compound A anion was prepared from Compound A (69.9 g, 0.15 mole) and NaH (6 g as 60% oil suspension, 0.15 mole) in DMF (180 ml)/THF (30 ml) mixed solvent as described in Example 2. To this solution was added 1-iodo-n-hexane (31.8 g, 0.15 mole)/THF (30 ml) solution, then the mixture was heated at 70°C for 24 hrs. The reaction mixture was cooled and worked up as in Example 2 to give the title product as a clear, colorless liquid, yield 34.8 g (42.3% yield). Bp. 80°C/0.75 mm. H¹NMR (CDCl₃): δ3.85 (t, J = 14 Hz, 2H), 3.58 (t, J = 6.5 Hz, 2H), 1.60 (m, 2H), 1.32 (m, br, 6H), 0.92 (t, J = 6.5 Hz, 3H); F¹⁹NMR (CDCl₃): -71.2 (q, J = 7 Hz, 2F), -77.3 (m, 1F), -80.2 (m, 3F), -82.2 to -85.8 (m, 4F), -123.6 (tm, J = 14 Hz, 2F), -146.2 (q, J = 20 Hz, 1F).

### EXAMPLE 4

### Preparation of 3,6,10-Trioxa-1,2-dichloro-1,1,2,4,4,5,7,7,8,8-decafluoro-5-trifluoromethyloctacosane

This compound was prepared by the procedure of Example 2 from Compound A (23.3 g, 0.05 mole), NaH (2 g as 60% oil suspension, 0.05 mole) and C₁₈H₃₇I (18.2 g, 0.0478 mole) in THF (70 ml) in a similar procedure as described in Example 3. The desired compound was a clear, viscous liquid, yield 8.1 g (23.5%). Bp. 140°C/0.012 mm. H¹NMR (CDCl₃): δ3.85 (t, J = 13.5 Hz, 2H), 3.56 (t, J = 7.0 Hz, 2H), 1.57 (m, 2H), 1.27 (s, br, 30H), 0.89 (m, 3H); F¹⁹NMR (CDCl₃): -71.2 (m, 2F), -77.4 (m, 1F), -80.2 (m, 3F), -82.5 to -86.0 (m, 4F), -123.6 (tm, J = 13.4 Hz, 2F), -146.2 (q, br, J = 21.1 Hz, 1F).

### EXAMPLE 5

### Preparation of 4,8,11-Trioxa-12,13-dichloro-6,6,7,7,9,10,10,12,13,13-decafluoro-9-trifluoromethyltridec-1-yne

This compound was prepared by the procedure of Example 2 from Compound A (23.3 g, 0.05 mole), NaH (2 g as 60% oil suspension, 0.05 mole) and propargyl bromide (7.14 g, 0.06 mole) in DMF (60 ml)/THF (10 ml) mixed solvent. The desired compound was obtained as a clear liquid, yield 10.8 g (43%). Bp. 104°C/20 mm. H¹NMR (CDCl₃): δ4.30 (d, J = 2.5 Hz, 2H), 4.00 (t, J = 13.5 Hz, 2H), 2.52 (t, J = 2.5 Hz, 1H); F¹⁹NMR (CDCl₃): -71.2 (m, 2F), -77.4 (m, 1F), -80.2 (m, 3F), -82.4 to -86.0 (m, 4F), -123.4 (tt, J = 1.7 Hz, 13.5 Hz, 2F), -146.2 (q, J = 19.8 Hz, 1F).

### EXAMPLE 6

### Preparation of 3,6,10-Trioxa-1,2-dichloro-1,1,2,4,4,5,7,7,8,8-decafluoro-5-trifluoro-methyl-11-(vinylphenyl)undecane

The title compound was prepared by the procedure of Example 2 from Compound A (23.3 g, 0.05 mole), NaH (2 g as 60% oil suspension, 0.05 mole) and vinyl benzylchloride (7.6 g, 0.05 mole, mixed isomers, purchased from Dow Chemical Co.) in DMF (65 ml)/THF (10 ml) mixed solvent at 80-85°C. Workup and distillation afforded 19.7 g (68% yield) of the pale-yellow clear liquid. Bp. 95-99°C/0.5 mm. H¹NMR (CDCl₃): δ7.42 to 7.20 (m, 4H), 6.62 (m, 1H), 5.75 (2s, 1H), 5.28 (2d, J = 12 Hz, 1H), 4.64 (2s, 2H), 3.88 (2t, J = 13.5 Hz, 2H); F¹⁹NMR (CDCl₃): -71.2 (m, 2F), -77.4 (m, 1F), -80.2 (m, 3F), -82.4 to -86.0 (m, 4F), -123.3 (t, J = 13.4 Hz, 2F), -146.2 (qm, J = 18.8 Hz, 1F).

### EXAMPLE 7

### Preparation of (3,6-Dioxa-1,2-dichloro-1,1,2,4,4,5,7,7,8,8-decafluoro-5-trifluoromethylnonyl) 2,4-Dinitrophenyl Ether

This compound was made by the procedure of Example 2 from Compound A (46.5 g, 0.010 mole), NaH (4 g as 60% oil suspension, 0.10 mole) and 2,4-dinitrofluorobenzene (27.9 g, 0.15 mole) in THF (150 ml). The final product was purified by distillation to give a liquid product, yield 45.0 g (71.3%). Bp. 150-160°C/0.5 mm. H¹NMR (CDCl₃): δ8.80 (d, J = 2.5 Hz, 1H), 8.70 (dd, J = 2.5, 10 Hz, 1H), 7.24 (d, J = 10 Hz, 1H), 4.68 (t, J = 12 Hz, 2H); F¹⁹NMR (CDCl₃): -71.3 (d, J = 10.1 Hz, 2F), -77.4 (m, 1F), -80.2 (q, J = 7.6 Hz, 3F), -82.5 to -86.0 (m, 4F), -122.9 (t, J = 11.6 Hz, 2F), -145.9 (q, J = 19.4 Hz, 1F).

### EXAMPLE 8

### Preparation of (3,6-Dioxa-1,2-dichloro-1,1,2,4,4,5,7,7,8,8-decafluoro-5-trifluoromethylnonyl) 2,4-Diaminophenyl Ether

The dinitro compound obtained from Example 7 (22.1 g, 0.035 mole) was mixed with 10% Pd/C (2.2 g) in ethyl acetate/ethanol (6/4 by volume, 88 g) solvent. Hydrogenation was carried out with 50-60 psi of hydrogen pressure at ambient temperature for about 6 hrs. After reaction, the solution was filtered to remove the metal residue and the solvent was removed in vacuo. The final diamino product was purified by distillation, 4.75 g of (24% yield) clear, viscous liquid was obtained. Bp. 120°C/0.02 mm. H¹NMR (CDCl₃): δ7.61 (d, J = 8.0 Hz, 1H), 6.08 (d, J = 2.0 Hz, 1H), 6.02 (dd, J = 2 Hz, 8 Hz, 1H), 4.11 (t, J = 13.5 Hz, 2H), 3.55 (s, br, 4H, -NH₂); F¹⁹NMR (CDCl₃): -71.3 (m, 2F), -77.5 (m, 1F), -80.2 (m, 3F), -82.3 to -86.0 (m, 4F), -124.0 (m, 2F), -146.2 (m, 1F).

### EXAMPLE 9

### Preparation of 3,6,10-Trioxa-5-trifluoromethyl-1,1,2,4,4,5,7,7,8,8-decafluorohexadec-1-ene

Zinc dust (7.80 g, 0.12 mole) in DMF (60 ml) solvent was activated with bromine (0.1 ml) and was heated at 90-95°C. The substrate obtained from Example 3 (32.96 g, 0.06 mole) was added and the reaction proceeded for 24 hrs. After cooling, ether was added to extract the product. The ether layer was washed with water, and dried over magnesium sulfate. The residue was distilled after ether solvent was removed in vacuo, to give 23.2 g (81% yield) of the desired product as a clear, colorless liquid. Bp. 75°C/3 mm. H¹NMR (CDCl₃): δ3.86 (t, J = 13.5 Hz, 2H), 3.55 (t, J = 6.6 Hz, 2H), 1.60 (m, 2H), 1.33 (m, br, 6H), 0.88 (t, J = 6.5 Hz, 3H); F¹⁹NMR (CDCl₃): -80.3 (t, J = 4.7 Hz, 3F), -84.1 (m, br, 2F), -85.2 (m, br, 2F), -123.6 (t, J = 13.5 Hz, 2F), -145.8 (t, J = 21.8 Hz, 1F), -113.5, -113.8, -113.9, -114.3 (4s, 1F), -121.6, -122.1, -122.2, -122.7 (4t, J = 5.3 Hz, 1F), -135.2, -135.6, -135.8, -136.2 (4t, J = 5.7 Hz, 1F).

### EXAMPLE 10

### Preparation of 3,6,10-Trioxa-5-trifluoromethyl-1,1,2,4,4,5,7,7,8,8-decafluorooctacos-1-ene

This compound was prepared from the product of Example 4 and zinc dust (1.64 g, 0.025 mole) in DMF solvent (12 ml) as described in Example 9. After work-up, the desired product 3.42 g (48.1% yield) was obtained as a pale-yellow liquid. Bp. 135°C/0.02 mm. H¹NMR (CDCl₃): δ3.86 (t, J = 13.5 Hz, 2H), 3.55 (t, J = 7.2 Hz, 2H), 1.57 (m, 2H), 1.28 (s, br, 30H), 0.88 (m, 3H); F¹⁹NMR (CDCl₃): -80.4 (m, 3F), -84.2 (m, 2F), -85.3 (m, 2F), -123.6 (t, J = 13.5 Hz, 2F), -145.8 (t, J = 21.3 Hz, 1F), -113.5, -113.9, -114.0, -114.3 (4s, 1F), -121.7, -122.1, -122.3, -122.7 (4t, J = 5.2 Hz, 1F), -135.2, -135.6, -135.8, -136.1 (4t, J = 5.7 Hz, 1F).

### EXAMPLE 11

### Preparation of 4,8,11-Trioxa-9-trifluoromethyl-6,6,7,7,9,10,10,12,13,13-decafluorotridec-12-ene-1-yne

The title compound was prepared from 10.1 g, 0.02 mole of the product of Example 5 and zinc dust (2.6 g, 0.04 mole) in DMF solvent (20 ml) as described in Example 9. The desired product was obtained as a clear, colorless liquid, yield 6.74 g (78%). Bp. 100°C/65 mm. H¹NMR (CDCl₃): δ4.30 (d, J = 2.5 Hz, 2H), 4.00 (t, J = 13.5 Hz, 2H), 2.51 (t, J = 2.5 Hz, 1H); F¹⁹NMR (CDCl₃): -80.3 (s, br, 3F), -84.2 (s, br, 2F), -85.3 (m, br, 2F), -123.3 (t, J = 13.3 Hz, 2F), -145.7 (t, J = 21.8 Hz, 1F), -113.4, -113.7, -113.8, -114.2 (4s, 1F), -121.6, -122.0, -122.2, -122.6 (4t, J = 5.1 Hz, 1F), -135.2, -135.6, -135.8, -136.2 (4t, J = 5.7 Hz, 1F).

### EXAMPLE 12

### Preparation of 3,6,10-Trioxa-5-trifluoromethyl-1,1,2,4,4,5,7,7,8,8-decafluoro-11-(vinylphenyl)undec-1-ene

This compound was made from 11.62 g, 0.02 mole, of the product of Example 6 and zinc dust (2.6 g, 0.04 mole) in DMF solvent (20 ml). After the same workup procedure as Example 9, the desired compound 4.37 g (43% yield) was obtained as a pale-yellow liquid. Bp. 85°C/0.9 mm. H¹NMR (CDCl₃): δ7.48 to 7.20 (m, 4H), 6.72 (m, 1H), 5.76 (2s, 1H), 5.27 (2d, J = 10.8 Hz, 1H), 4.63 (2s, 2H), 3.88 (2t, J = 13.5 Hz, 2H); F¹⁹NMR (CDCl₃): -80.3 (m, 3F), -84.2 (m, 2F), -85.2 (m, 2F), -123.2 (t, J = 13.4 Hz, 2F), -145.7 (t, J = 21.8 Hz, 1F), -113.3, -113.7, -113.8, -114.1 (4s, 1F), -121.5, -122.0, -122.1, -122.6 (4t, J = 5.4 Hz, 1F), -135.2, -135.5, -135.8, -136.1 (4t, J = 5.7 Hz, 1F).

### EXAMPLE 13

### Preparation of 4,7-Dioxa-5-trifluoromethyl-2,2,3,3,5,6,6,8,9,9-decafluoronon-8-enyl Methacrylate

To a dry flask was charged methyl 4,7-dioxa-5-trifluoromethyl-2,2,3,3,5,6,8,9,9-decafluoronon-8-enoate (211 g, 0.50 mole) in absolute ethanol (300 ml) with a magnetic stirring bar. Sodium borohydride (11.34 g, 0.30 mole) was added slowly from a solid addition funnel. The reaction was somewhat exothermic and the reaction pot was kept at ≦ 10°C by external cooling. After the addition of sodium borohydride was completed, the reaction mixture was stirred for 1 hr at room temperature. The pot mixture was then dumped into an ice water (600 ml)/6N HCl (600 ml) mixture. The bottom product layer was separated, washed with water and distilled to give the corresponding alcohol as a clear, colorless liquid. Bp. 68°C/25 mmHg. Yield: 168.7 g (85.6%).

The alcohol was dissolved in methylene chloride at 0-5°C in the presence of pyridine (5.93 g, 0.075 mole). Methacrylyl chloride (8.7 g, 90% purity, 0.075 mole, purchased from Aldrich Chemical Co.) was added slowly, and the mixture was allowed to stir 1 hr at 5°C after the addition. The mixture was then dumped into ice water, the bottom organic layer was separated, washed with 5% HCl and water, and dried over magnesium sulfate. The solvent was removed in vacuo, and the residue was distilled to give the desired product as a clear, colorless liquid, yield 17.3 g (74.8%). Bp. 75°C/5 mm. H¹NMR (CDCl₃): δ6.21, 6.24 (2s, br, 1H), 5.70, 5.82 (2s, br, 1H), 4.59 (t, J = 13.5 Hz, 2H), 1.98, 2.02 (2s, br, 3H); F¹⁹NMR (CDCl₃): -80.4 (m, 3F), -84.3 (m, 2F), -85.3 (m, 2F), -123.4 (t, J = 13.0 Hz, 2F), -145.8 (t, J = 21.6 Hz, 1F), -113.3, -113.6, -113.7, -114.1 (4s, 1F), -121.5, -121.9, -122.1, -122.5 (4t, J = 5.6 Hz, 1F), -135.3, -135.6, -135.9, -136.2 (4t, J = 5.8 Hz, 1F).

### EXAMPLE 14

### Free Radical Copolymerization of Tetrafluoroethylene and 3,6,10-Trioxa-5-trifluoromethyl-1,1,2,4,4,5,7,7,8,8-decafluorohexadec-1-ene

The monomer (2 g) prepared as described in Example 9 was dissolved in 1,1,2-trichloro-1,2,2-trifluoroethane (10 g) in the presence of 4,4'-bis(t-butylcyclohexyl)peroxy dicarbonate initiator (0.01 g) in a 75 ml stainless steel tube. This tube was pressurized with tetrafluoroethylen to 276 kPa (40 psi) at room temperature. The tube was then sealed and was heated at 50°C, 70°C and 90°C for 2 hrs each respectively. Solvent was removed from the resulting polymer which was then washed with water, acetone and ether, dried under vacuum (150 mm) at 100°C for 24 hrs. White polymer (3.3 g) was obtained, which had a Tg at 180°C and two Tm's at 258 and 320°C respectively. The composition of this polymer was TFE/CF₂=CFOCF₂CF(CF₃)O-CF₂CF₂CH₂O-C₆H₁₃ = 93.6/6.4 (mole %) by F¹⁹ high temperature NMR spectroscopy.

### EXAMPLE 15

### Free Radical Homopolymerization of 4,7-Dioxa-5-trifluoromethyl-2,2,3,3,5,6,6,8,9,9-decafluoronon-8-enyl Methacrylate

The title monomer (3.0 g) and benzoyl peroxide (0.01 g) were sealed in a vial with a stirrer under an inert atmosphere. The polymerization proceeded at 65°C for 70 hrs, then at 100°C for 8 hrs. At this time a tough, transparent and white solid polymer was obtained in quantitative yield.

### EXAMPLE 16

### Free Radical Homopolymerization of 3,6,10-Trioxa-5-trifluoromethyl-1,1,2,4,4,5,7,7,8,8-decafluoro-11-(vinylphenyl)undec-1-ene

The title monomer (2.0 g) and benzoyl peroxide (0.01 g) were sealed in a reaction vial. The polymerization was carried out at 65°C for 70 hrs. A hard polymer, 1.92 g, was obtained after washing and drying. This polymer had good resistance toward common organic solvents, such as acetone, chloroform, THF, etc.

## Claims

1. A trifluorovinyl ether comprising the formula I
CF₂=CF-[OCF₂CF(CF₃)]ₚ-[O(CF₂)_{q}]ᵣ-CH₂OR¹ I
wherein:
R¹ is alkyl or cycloalkyl containing 1 to 30 carbon atoms, 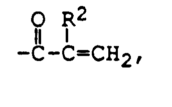 -(CH₂)ₛR³ or -(CH₂)ₜR⁵;
R² is hydrogen or methyl;
R³ is -C≡CH, -CH=CH₂, styryl, or 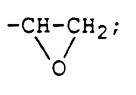 R⁵ is aryl or substituted aryl;
p is an integer of 0 to 20;
q is an integer of 1 to 10;
r is 0 or 1;
s is 1, 2, 3 or 4; and
t is 0, 1, 2, 3 or 4;
provided that when r is 0, p is not 0.

2. The trifluorovinyl ether of Claim 1 wherein said p is 0, said r is 1, and said q is 1 to 10.

3. The trifluorovinyl ether of Claim 1 wherein said p is 1, said r is 1, and said q is 2.

4. The trifluorovinyl ether of Claim 1 wherein said R¹ group is:
n-alkyl;
-(CH₂)ₛR³ wherein s is 1, and R³ is -C≡CH, -CH=CH₂, styryl, or 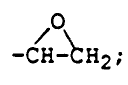 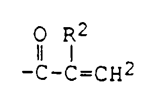 wherein R² is hydrogen or methyl; or -(CH₂)ₜR⁵ wherein t is 0 or 1.

5. The trifluorovinyl ether of Claim 4 wherein said R¹ group is -(CH₂)ₛR³ wherein s is 1 and R³ is styryl, -CH=CH₂ or 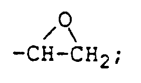 or said R¹ group is 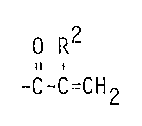 wherein R² is hydrogen or methyl.

6. The trifluorovinyl ether of Claim 1 wherein said t is 0, and said substituted aryl is substituted with, or has substituents derived from, electron withdrawing groups.

7. The trifluorovinyl ether of Claim 4 wherein said p is 0, said r is 1, and said q is 2.

8. The trifluorovinyl ether of Claim 4 wherein said p is 1, said r is 1 and said q is 2.

9. The trifluorovinyl ether of Claim 1 which is:
3,6,10-trioxa-5-trifluoromethyl-1,1,2,4,4,5,7,7,8,8-decafluorohexadec-1-ene;
3,6,10-trioxa-5-trifluoromethyl-1,1,2,4,4,5,7,7,8,8-decafluorooctacos-1-ene;
4,8,11-trioxa-9-trifluoromethyl-6,6,7,7,9,10,10,12,13,13-decafluorotridec-12-ene-1-yne;
3,6,10-trioxa-5-trifluoromethyl-1,1,2,4,4,5,7,7,8,8-decafluoro-11-(vinylphenyl)undec-1-ene; or
4,7-dioxa-5-trifluoromethyl-2,2,3,3,5,6,6,8,9,9-decafluoronon-8-enyl methacrylate.

10. A halogenated compound comprising the formula II
CF₂XCFX-[OCF₂CF(CF₃)]ₚ-[O(CF₂)_{q}]ᵣ-CH₂OR¹ II
wherein:
R¹ is alkyl or cycloalkyl containing 1 to 30 carbon atoms, 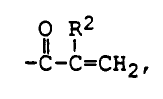 -(CH₂)ₛR³, -(CH₂)ₜR⁵ or hydrogen;
R² is hydrogen or methyl;
R³ is -C≡CH, -CH=CH₂, styryl, or 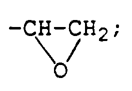 R⁵ is aryl, or substituted aryl;
each X is independently chlorine or bromine;
p is an integer of 0 to 20;
q is an integer of 1 to 10;
r is 0 or 1;
s is 1, 2, 3 or 4; and
t is 0, 1, 2, 3, or 4;
provided that when r is 0, p is not 0, and further provided that when p is 0, q is three, r is 1, and R¹ is not hydrogen.

11. The halogenated compound of Claim 10 wherein said p is 0, said r is 1, and said q is 1 to 10.

12. The halogenated compound of Claim 10 wherein said p is 1, said r is 1, and said q is 2.

13. The halogenated compound of Claim 10 wherein said R¹ is:
n-alkyl;
-(CH₂)ₛR³ wherein s is 1, and R³ is -C≡CH, -CH=CH₂ or styryl; or
-(CH₂)ₜR⁵ wherein t is 0 or 1.

14. The halogenated compound of Claim 13 wherein said R¹ group is -CH=CH₂ or styryl.

15. The halogenated compound of Claim 13 wherein said p is 0, said r is 1, and said q is 1 to 10.

16. The halogenated compound of Claim 13 wherein said p is 1, said r is 1, and said q is 2.

17. The halogenated compound of Claim 10 wherein said t is 0, and said substituted aryl is substituted with, or has substituents derived from, electron withdrawing groups.

18. The halogenated compound of Claim 10 which is:
3,6,10-trioxa-1,2-dichloro-1,1,2,4,4,5,7,7,8,8-decafluoro-5-trifluoromethyl-12,13-epoxy-tridecane;
3,6,10-trioxa-1,2-dichloro-1,1,2,4,4,5,7,7,8,8-decafluoro-5-trifluoromethylhexadecane;
3,6,10-trioxa-1,2-dichloro-1,1,2,4,4,5,7,7,8,8-decafluoro-5-trifluoromethyloctacosane;
4,8,11-trioxa-12,13-dichloro-6,6,7,7,9,10,10,12,13,13-decafluoro-9-trifluoromethyltridec-1-yne;
3,6,10-trioxa-1,2-dichloro-1,1,2,4,4,5,7,7,8,8-decafluoro-5-trifluoromethy-11-(vinylphenyl)undecane;
(3,6-dioxa-1,2-dichloro-1,1,2,4,4,5,7,7,8,8-decafluoro-5-trifluoromethylnonyl) 2,4-dinitrophenyl ether; or
(3,6-dioxa-1,2-dichloro-1,1,2,4,4,5,7,7,8,8-decafluoro-5-trifluoromethylnonyl) 2,4-diaminophenyl ether.

19. A copolymer of a trifluorovinyl ether comprising units of the formula III 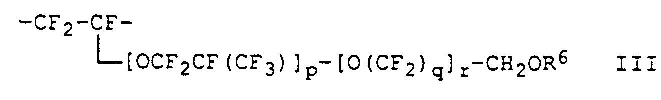 and one or more other monomers wherein:
R⁶ is alkyl or cycloalkyl containing 1 to 30 carbon atoms, -(CH₂)ₛR⁸, or -(CH₂)ₜR⁵;
R⁸ is -C≡CH, -CH=CH₂, or 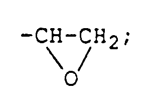 R⁵ is aryl, or substituted aryl;
p is an integer of 0 to 20;
q is an integer of 1 to 10;
r is 0 or 1;
s is 1, 2, 3 or 4; and
t is 0, 1, 2, 3, or 4;
provided that when r is 0, p is not 0.

20. The copolymer of Claim 19 wherein said p is 0, said r is 1 and said q is 1 to 10.

21. The copolymer of Claim 19 wherein said p is 1, said r is 1 and said q is 2.

22. The copolymer as recited in claim 19 wherein said R¹ is:
n-alkyl;
-(CH₂)ₛR³ wherein s is 1 and R³ is -C≡CH, -CH=CH₂, or 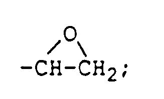 or
-(CH₂)ₜR⁵ wherein t is 0 or 1.

23. The copolymer of Claim 19 wherein said t is 0, said R⁵ is substituted with, or have substituents derived from, electron withdrawing groups.

24. The copolymer of Claim 22 wherein said p is 0, said r is 1 and said q is 1 to 10.

25. The copolymer of Claim 22 wherein said p is 1, said r is 1 and said q is 2.

26. The copolymer of Claim 19 which is a copolymer with a vinyl monomer or ethylene.

27. The copolymer of Claim 26 wherein said vinyl monomer is a fluorinated vinyl monomer.

28. The copolymer of Claim 27 wherein said fluorinated vinyl monomer is vinylidene fluoride, tetrafluoroethylene, vinyl fluoride, perfluoro(methyl vinyl ether), chlorotrifluoroethylene, perfluoro-1-heptene, hexafluoropropylene, or perfluoro-2,2-dimethyl-1,3-dioxole.

29. The copolymer of Claim 28 wherein said fluorinated vinyl monomer is tetrafluoroethylene.

30. The copolymer of Claim 24 which is a copolymer with a fluorinated vinyl monomer.

31. The copolymer of Claim 25 which is a copolymer with a fluorinated vinyl monomer.

32. The copolymer of Claim 30 wherein said fluorinated vinyl monomer is vinylidene fluoride, tetrafluoroethylene, vinyl fluoride, perfluoro(methyl vinyl ether), chlorotrifluoroethylene, perfluoro-1-heptene, hexafluoropropylene, or perfluoro-2,2-dimethyl-1,3-dioxole.

33. The copolymer of Claim 31 wherein said fluorinated vinyl monomer is vinylidene fluoride, tetrafluoroethylene, vinyl fluoride, perfluoro(methyl vinyl ether), chlorotrifluoroethylene, perfluoro-1-heptene, hexafluoropropylene, or perfluoro-2,2-dimethyl-1,3-dioxole.

34. The copolymer of Claim 19 wherein said units of the formula III 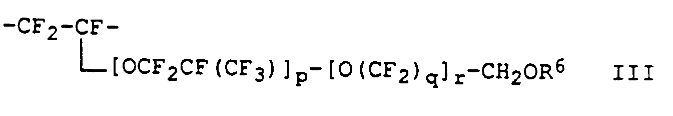 are about 20 mole percent or less of the polymer.

35. The copolymer of Claim 32 wherein said units of the formula III 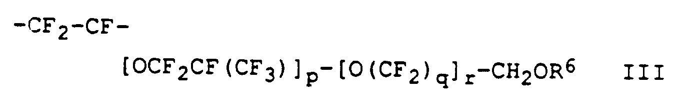 are about 20 mole percent or less of the polymer.

36. A polymer made by the free radical polymerization of:
1) a compound of the formula IV
CF₂=CF-[OCF₂CF(CF₃)]ₚ-[O(CF₂)_{q}]ᵣ-CH₂OR⁷ IV
or
2) a compound of formula IV and other vinyl monomers,
wherein:
R⁷ is 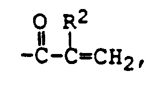 or -(CH₂)ₛR⁴;
R² is hydrogen or methyl;
R⁴ is styryl;
p is an integer of 0 to 20;
q is an integer of 1 to 10;
r is 0 or 1; and
s is 1, 2, 3 or 4;
provided that when r is 0, p is not 0.

37. A polymer of Claim 36 wherein said p is 0, said r is 1 and said q is 1 to 10.

38. A polymer of Claim 36 wherein said p is 1, said r is 1 and said q is 2.

39. A polymer of Claim 36 wherein said s is 1.

40. A polymer of Claim 37 wherein said s is 1.

41. A polymer of Claim 38 wherein said s is 1.

42. A polymer of Claim 36 wherein said compound of the formula IV
CF₂=CF-[OCF₂CF(CF₃)]ₚ-[O(CF₂)_{q}]ᵣ-CH₂OR⁷ IV
is about 20 mole percent or less of the total number of monomer units in the copolymer.

43. The polymer of Claim 36 which is a copolymer with one or more of acrylic esters, acrylamides, styrene, vinyl acetate, propylene, tetrafluoroethylene, chlorotrifluoroethylene, vinyl fluoride, vinylidene fluoride, hexafluoropropylene, or perfluoro-2,2-dimethyl-1,3-dioxole.

## Patentansprüche

1. Trifluorvinylether, umfassend die Formel I
CF₂=CF-[OCF₂CF(CF₃)]ₚ-[O(CF₂)_{q}]ᵣ-CH₂OR¹ I,
worin
R¹ für Alkyl oder Cycloalkyl steht, enthaltend 1 bis 30 Kohlenstoffatome, 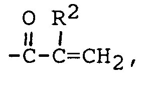 -(CH₂)ₛR³ oder -(CH₂)ₜR⁵,
R² für Wasserstoff oder Methyl steht,
R³ für -C≡CH, -CH=CH₂, Styryl oder 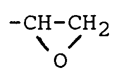 steht,
R⁵ für Aryl oder substituiertes Aryl steht,
p eine ganze Zahl von 0 bis 20 bedeutet,
q eine ganze Zahl von 1 bis 10 bedeutet,
r für 0 oder 1 steht,
s 1, 2, 3 oder 4 bedeutet und
t 0, 1, 2, 3 oder 4 bedeutet,
mit der Maßgabe, daß, wenn r nicht für 0 steht, p nicht für 0 steht.

2. Trifluorvinylether nach Anspruch 1, worin das genannte p 0 bedeutet, das genannte r 1 bedeutet und das genannte q 1 bis 10 bedeutet.

3. Trifluorvinylether nach Anspruch 1, worin das genannte p 1 bedeutet, das genannte r 1 bedeutet und das genannte q 2 bedeutet.

4. Trifluorvinylether nach Anspruch 1, worin die genannte Gruppe R¹ für folgendes steht:
n-Alkyl,
-(CH₂)ₛR³, worin s für 1 steht und R³ für -C≡CH, -CH=CH₂, Styryl oder 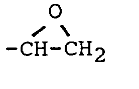 steht, 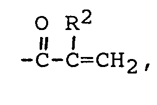 worin R² für Wasserstoff oder Methyl steht, oder
-(CH₂)ₜR⁵, worin t 0 oder 1 bedeutet.

5. Trifluorvinylether nach Anspruch 4, worin die genannte Gruppe R¹ -(CH₂)ₛR³ bedeutet, worin s für 1 steht und R³ für Styryl, -CH=CH₂ oder 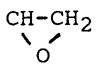 steht, oder die genannte Gruppe R¹ für 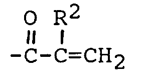 steht, worin R² für Wasserstoff oder Methyl steht.

6. Trifluorvinylether nach Anspruch 1, worin das genannte t für 0 steht und das genannte substituierte Aryl mit elektronenziehenden Gruppen substituiert ist oder Substituenten aufweisen, die sich von einer elektronenziehendenen Gruppen ableiten.

7. Trifluorvinylether nach Anspruch 4, worin das genannte p 0 bedeutet, das genannte r 1 bedeutet und das genannte q 2 bedeutet.

8. Trifluorvinylether nach Anspruch 4, worin das genannte p 1 bedeutet, das genannte r 1 bedeutet und das genannte q 2 bedeutet.

9. Trifluorvinylether nach Anspruch 1, der folgendermaßen heißt:
3,6,10-Trioxa-5-trifluormethyl-1,1,2,4,4,5,7,7,8,8-decafluorhexadec-1-en,
3,6,10-Trioxa-5-trifluormethyl-1,1,2,4,4,5,7,7,8,8-decaflourctacos-1-en,
4,8,11-Trioxa-9-trifluormethyl-6,6,7,7,9,10,10,12,13,13-decafluortridec-12-en-1-in,
3,6,10-Trioxa-5-trifluormethyl-1,1,2,4,4,5,7,7,8,8-decafluor-11-(vinylphenyl)-undec-1-en, oder
4,7-Dioxa-5-triflormethyl-2,2,3,3,5,6,6,8,9,9-decafluornon-8-enyl-methacrylat.

10. Halogenierte Verbindung, umfassend die Formel II:
CF₂XCFX-[OCF₂CF(CF₃)]ₚ-[O(CF₂)_{q}]ᵣ-CH₂OR¹ II,
worin
R¹ für Alkyl oder Cycloalkyl, enthaltend 1 bis 30 Kohlenstoffatome, 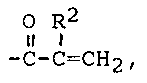 -(CH₂)ₛR³ oder -(CH₂)ₜR⁵, oder Wasserstoff steht,
R² für Wasserstoff oder Methyl steht,
R³ für -C≡CH, -CH=CH₂, Styryl oder 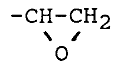 steht,
R⁵ für Aryl oder substituiertes Aryl steht,
X jeweils unabhängig für Chlor oder Brom steht,
p eine ganze Zahl von 0 bis 20 bedeutet,
q eine ganze Zahl von 1 bis 10 bedeutet,
r 0 oder 1 bedeutet,
s 1, 2, 3 oder 4 bedeutet und
t 0, 1, 2, 3 oder 4 bedeutet,
mit der Maßgabe, daß, wenn r für 0 steht, p nicht für 0 steht und außerdem mit der Maßgabe, daß, wenn p für 0 steht, q 3 bedeutet, r 1 bedeutet und R¹ nicht für Wasserstoff steht.

11. Halogenierte Verbindung nach Anspruch 10, worin das genannte p 0 bedeutet, das genannte r 1 bedeutet und das genannte q 1 bis 10 bedeutet.

12. Halogenierte Verbindung nach Anspruch 10, worin das genannte p 1 bedeutet, das genannte r 1 bedeutet und das genannte q 2 bedeutet.

13. Halogenierte Verbindung nach Anspruch 10, worin das genannte R¹ für folgendes steht:
n-Alkyl,
-(CH₂)ₛR³, worin s 1 bedeutet und R³ für -C≡CH, -CH=CH₂ oder Styryl steht, oder
-(CH₂)ₜR⁵, worin t für 0 oder 1 steht.

14. Halogenierte Verbindung nach Anspruch 13, worin die genannte Gruppe R¹ für -CH=CH₂ oder Styryl steht.

15. Halogenierte Verbindung nach Anspruch 13, worin das genannte p für 0 steht, das genannte r 1 bedeutet und das genannte q 1 bis 10 bedeutet.

16. Halogenierte Verbindung nach Anspruch 13, worin das genannte p 1 bedeutet, das genannte r 1 bedeutet und das genannte q 2 bedeutet.

17. Halogenierte Verbindung nach Anspruch 10, worin das genannte t 0 bedeutet und das genannte substituierte Aryl mit elektronenziehendenen Gruppen substituiert ist oder Substituenten aufweist, die sich davon ableiten.

18. Halogenierte Verbindung nach Anspruch 10, die folgendermaßen heißt:
3,6,10-Trioxa-1,2-dichlor-1,1,2,4,4,5,7,7,8,8-decafluor-5-trifluormethyl-12,13-epoxytridecan,
3,6,10-Trioxa-1,2-dichlor-1,1,2,4,4,5,7,7,8,8-decaflour-5-trifluormethylhexadecan,
3,6,10-Trioxa-1,2-dichlor-1,1,2,4,4,5,7,7,8,8-decafluor-5-trifluormethyloctacosan,
4,8,11-Trioxa-12,13-dichlor-6,6,7,7,9,10,10,12,13,13-decafluor-9-trifluormethyltridec-1-in,
3,6,10-Trioxa-1,2-dichlor-1,1,2,4,4,5,7,7,8,8-decafluor-5-trifluormethyl-11-(vinylphenyl)-undecan,
(3,6-Dioxa-1,2-dichlor-1,1,2,4,4,5,7,7,8,8-decafluor-5-trifluormethylnonyl)-2,4-dinitrophenylether, oder
(3,6-Dioxa-1,2,-dichlor-1,1,2,4,4,5,7,7,8,8-decafluor-5-trifluormethylnonyl)-2,4-diaminphenylether.

19. Copolymer von Trifluorvinylether, umfassend Einheiten der Formel III 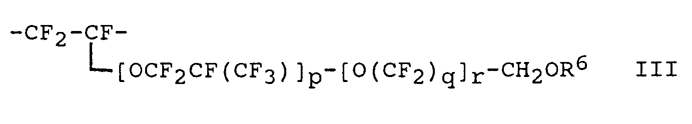 und eines oder mehrere weitere Monomere, worin
R⁶ für Alkyl oder Cycloalkyl, enthaltend 1 bis 30 Kohlenstoffatome, -(CH₂)ₛR⁸ oder -(CH₂)ₜR⁵ steht,
R⁸ für -C≡CH, -CH=CH₂, oder 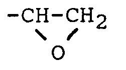 steht,
R⁵ für Aryl oder substituiertes Aryl steht,
p eine ganze Zahl von 0 bis 20 bedeutet,
q eine ganze Zahl von 1 bis 10 bedeutet,
r 0 oder 1 bedeutet,
s 1, 2, 3 oder 4 bedeutet und
t 0, 1, 2, 3 oder 4 bedeutet,
mit der Maßgabe, daß wenn r für 0 steht, p nicht für 0 steht.

20. Copolymer nach Anspruch 19, worin das genannte p für 0 steht, das genannte r für 1 steht und das genannte q für 1 bis 10 steht.

21. Copolymer nach Anspruch 19, worin das genannte p für 1 steht, das genannte r für 1 steht und das genannte q für 2 steht.

22. Copolymer nach Anspruch 19, worin das genannte R¹ für folgendes steht:
n-Alkyl,
-(CH₂)ₛR³, worin s für 1 steht und R³ für -C≡CH, -CH=CH₂, Styryl oder 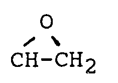 steht, oder
-(CH₂)ₜR⁵, worin t für 0 oder 1 steht.

23. Copolymer nach Anspruch 19, worin das genannte t für 0 steht, das genannte r mit elektronenziehendenen Gruppen substituiert ist oder Substituenten aufweist, die sich davon ableiten.

24. Copolymer nach Anspruch 22, worin das genannte p für 0 steht, das genannte r für 1 steht und das genannte q 1 bis 10 bedeutet.

25. Copolymer nach Anspruch 22, worin das genannte p für 1 steht, das genannte r für 1 steht und das genannte q für 2 steht.

26. Copolymer nach Anspruch 19, das ein Copolymer mit einem Vinyl-Monomer oder mit Ethylen ist.

27. Copolymer nach Anspruch 26, worin das genannte Vinyl-Monomer ein fluoriertes Vinyl-Monomer ist.

28. Copolymer nach Anspruch 27, worin das genannte fluorierte Vinyl-Monomer Vinylidenfluorid, Tetrafluorethylen, Vinylfluorid, Perfluor(methylvinyl)-ether, Chlortrifluorethylen, Perfluor-1-hepten, Hexafluorpropylen oder Perfluor-2,2-dimethyl-1,3-dioxol ist.

29. Copolymer nach Anspruch 28, worin das genannte fluorierte Vinyl-Monomer Tetrafluorethylen ist.

30. Copolymer nach Anspruch 24, das ein Copolymer mit einem fluorierten Vinyl-Monomer ist.

31. Copolymer nach Anspruch 25, das ein Copolymer mit einem fluorierten Vinyl-Monomer ist.

32. Copolymer nach Anspruch 30, worin das genannte fluorierte Vinyl-Monomer für Vinylidenfluorid, Tetrafluorethylen, Vinylfluorid, Perfluor(methylvinyl)-ether, Chlortrifluorethylen, Perfluor-1-hepten, Hexafluorpropylen oder Perfluor-2,2-dimethyl-1,3-dioxol steht.

33. Copolymer nach Anspruch 31, worin das genannte fluorierte Vinyl-Monomer für Vinylidenfluorid, Tetrafluorethylen, Vinylfluorid, Perfluor(methylvinyl)-ether, Chlortrifluorethylen, Perfluor-1-hepten, Hexafluorpropylen oder Perfluor-2,2-dimethyl-1,3-dioxol steht.

34. Copolymer nach Anspruch 19, worin die genannten Einheiten der Formel III 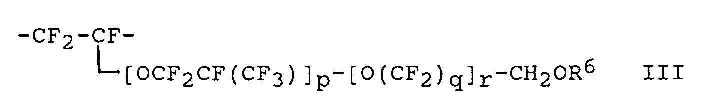 etwa 20 Mol-% oder weniger des Polymeren ausmachen.

35. Copolymer nach Anspruch 32, worin die genannten Einheiten der Formel III 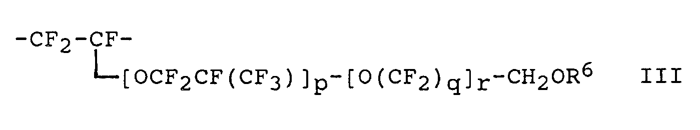 etwa 20 Mol-% oder weniger des Polymeren ausmachen.

36. Polymer, hergestellt durch frei-radikalische Polymerisation von
1) einer Verbindung der Formel IV
CF₂=CF-[OCF₂CF(CF₃)]ₚ-[O(CF₂)_{q}]ᵣ-CH₂OR⁷ IV
oder
2) einer Verbindung der Formel IV und weiteren Vinyl-Monomeren,
worin
R⁷ für 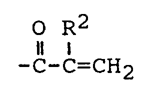 oder -(CH₂)ₛR⁴ steht,
R² für Wasserstoff oder Methyl steht,
R⁴ für Styryl steht,
p für eine ganze Zahl von 0 bis 20 steht,
q für eine ganze Zahl von 1 bis 10 steht,
r für 0 oder 1 steht und
s für 1, 2, 3 oder 4 steht,
mit der Maßgabe, daß, wenn r 0 bedeutet, p nicht 0 bedeutet.

37. Polymer nach Anspruch 36, worin das genannte p für 0 steht, das genannte r für 1 steht und das genannte q 1 bis 10 bedeutet.

38. Polymer nach Anspruch 36, worin das genannte p für 1 steht, das genannte r für 1 steht und das genannte q für 2 steht.

39. Polymer nach Anspruch 36, worin das genannte s für 1 steht.

40. Polymer nach Anspruch 37, worin das genannte s für 1 steht.

41. Polymer nach Anspruch 38, worin das genannte s für 1 steht.

42. Polymer nach Anspruch 36, worin die genannte Verbindung der Formel IV
CF₂=CF-[OCF₂CF(CF₃)]ₚ-[O(CF₂)_{q}]ᵣ-CH₂OR⁷ IV
etwa 20 Mol-% oder weniger der Gesamtanzahl der Monomer-Einheiten in dem Copolymer darstellt.

43. Polymer nach Anspruch 36, das ein Copolymer mit einem oder mehreren Acrylestern, Acrylamiden, Styrol, Vinylacetat, Propylen, Tetrafluorethylen, Chlortrifluorethylen, Vinylfluorid, Vinylidenfluorid, Hexafluorpropylen oder Perfluor-2,2-dimethyl-1,3-dioxol ist.

## Revendications

1. Un éther trifluorovinylique répondant à la formule I
CF₂=CF-[OCF₂CF(CF₃)]ₚ-[O(CF₂)_{q}]ᵣ-CH₂OR¹ I
dans laquelle :
R¹ est un groupe alkyle ou cycloalkyle contenant 1 à 30 atomes de carbone, 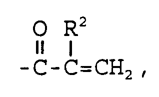 -(CH₂)ₛR³ ou -(CH₂)ₜR⁵ ;
R² est l'hydrogène ou un groupe méthyle ;
R³ est un groupe -C≡CH, -CH=CH₂, styryle ou 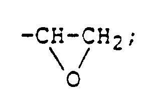 R⁵ est un groupe aryle ou aryle substitué ;
p est un nombre entier de 0 à 20 ;
q est un nombre entier de 1 à 10 ;
r est 0 ou 1 ;
s est 1, 2, 3 ou 4 ; et
t est 0, 1, 2, 3 ou 4 ;
à condition que si r est 0, p ne soit pas 0.

2. L'éther trifluorovinylique de la revendication 1, dans lequel ledit p est 0, ledit r est 1 et ledit q est de 1 à 10.

3. L'éther trifluorovinylique de la revendication 1, dans lequel ledit p est 1, ledit r est 1 et ledit q est 2.

4. L'éther trifluorovinylique de la revendication 1, dans lequel ledit groupe R¹ est :
*n*-alkyle ;
-(CH₂)ₛR³ où s est 1 et R³ est un groupe -C≡CH, -CH=CH₂, styryle ou 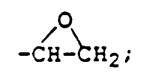 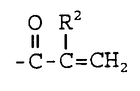 où R² est l'hydrogène ou un groupe méthyle ; ou
-(CH₂)ₜR⁵ où t est 0 ou 1.

5. L'éther trifluorovinylique de la revendication 4, dans lequel ledit groupe R¹ est -(CH₂)ₛR³ où s est 1 et R³ est un groupe styryle, -CH=CH₂ ou 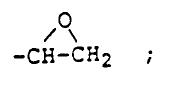 ou bien ledit groupe R¹ est 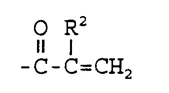 où R² est l'hydrogène ou un groupe méthyle.

6. L'éther trifluorovinylique de la revendication 1, dans lequel ledit t est 0, et ledit groupe aryle substitué est substitué par des groupes électro-attractifs ou porte des substituants dérivés de tels groupes.

7. L'éther trifluorovinylique de la revendication 4, dans lequel ledit p est 0, ledit r est 1 et ledit q est 2.

8. L'éther trifluorovinylique de la revendication 4, dans lequel ledit p est 1, ledit r est 1 et ledit q est 2.

9. L'éther trifluorovinylique de la revendication 1, qui est :
le 3,6,10-trioxa-5-trifluorométhyl-1,1,2,4,4,5,7,7,8,8-décafluorohexadéc-1-ène ;
le 3,6,10-trioxa-5-trifluorométhyl-1,1,2,4,4,5,7,7,8,8-décafluorooctacos-1-ène ;
le 4,8,11-trioxa-9-trifluorométhyl-6,6,7,7,9,10,10,12,13,13-décafluorotridéc-12-ène-1-yne ;
le 3,6,10-trioxa-5-trifluorométhyl-1,1,2,4,4,5,7,7,8,8-décafluoro-11-(vinylphényl)undéc-1-ène ; ou
le méthacrylate de 4,7-dioxa-5-trifluorométhyl-2,2,3,3,5,6,6,8,9,9-décafluoronon-8-ényle.

10. Un composé halogéné répondant à la formule II
CF₂XCFX-[OCF₂CF(CF₃)]ₚ-[O(CF₂)_{q}]ᵣ-CH₂OR¹ II
dans laquelle :
R¹ est un groupe alkyle ou cycloalkyle contenant 1 à 30 atomes de carbone, 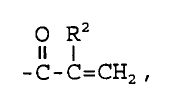 -(CH₂)ₛR³, -(CH₂)ₜR⁵ ou l'hydrogène ;
R² est l'hydrogène ou un groupe méthyle ;
R³ est un groupe -C≡CH, -CH=CH₂, styryle ou 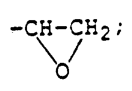 R⁵ est un groupe aryle ou aryle substitué ;
chaque X est indépendamment le chlore ou le brome ;
p est un nombre entier de 0 à 20 ;
q est un nombre entier de 1 à 10 ;
r est 0 ou 1 ;
s est 1, 2, 3 ou 4 ; et
t est 0, 1, 2, 3 ou 4 ;
à condition que si r est 0, p ne soit pas 0, et à condition encore que si p est 0, q soit 3, r soit 1 et R¹ ne soit pas l'hydrogène.

11. Le composé halogéné de la revendication 10, dans lequel ledit p est 0, ledit r est 1 et ledit q est de 1 à 10.

12. Le composé halogéné de la revendication 10, dans lequel ledit p est 1, ledit r est 1 et ledit q est 2.

13. Le composé halogéné de la revendication 10, dans lequel R¹ est un groupe :
*n*-alkyle ;
-(CH₂)ₛR³ où s est 1 et R³ est un groupe -C≡CH, -CH=CH₂ ou styryle ; ou
-(CH₂)ₜR⁵ où t est 0 ou 1.

14. Le composé halogéné de la revendication 13, dans lequel ledit groupe R¹ est -CH=CH₂ ou un groupe styryle.

15. Le composé halogéné de la revendication 13, dans lequel ledit p est 0, ledit r est 1 et ledit q est de 1 à 10.

16. Le composé halogéné de la revendication 13, dans lequel ledit p est 1, ledit r est 1 et ledit q est 2.

17. Le composé halogéné de la revendication 10, dans lequel ledit t est 0 et ledit groupe aryle substitué est substitué par des groupes électro-attractifs ou porte des substituants dérivés de tels groupes.

18. Le composé halogéné de la revendication 10, qui est :
le 3,6,10-trioxa-1,2-dichloro-1,1,2,4,4,5,7,7,8,8-décafluoro-5-trifluorométhyl-12,13-époxytridécane ;
le 3,6,10-trioxa-1,2-dichloro-1,1,2,4,4,5,7,7,8,8-décafluoro-5-trifluorométhylhexadécane ;
le 3,6,10-trioxa-1,2-dichloro-1,1,2,4,4,5,7,7,8,8-décafluoro-5-trifluorométhyloctacosane ;
le 4,8,11-trioxa-12,13-dichloro-6,6,7,7,9,10,10,12,13,13-décafluoro-9-trifluorométhyltridéc-1-yne ;
le 3,6,10-trioxa-1,2-dichloro-1,1,2,4,4,5,7,7,8,8-décafluoro-5-trifluorométhyl-11-(vinylphényl)undécane ;
l'éther de (3,6-dioxa-1,2-dichloro-1,1,2,4,4,5,7,7,8,8-décafluoro-5-trifluorométhylnonyle) et de 2,4-dinitrophényle ; ou
l'éther de (3,6-dioxa-1,2-dichloro-1,1,2,4,4,5,7,7,8,8-décafluoro-5-trifluorométhylnonyle) et de 2,4-diaminophényle.

19. Un copolymère d'un éther trifluorovinylique comprenant des motifs de la formule III 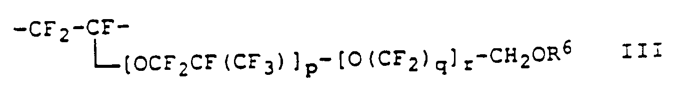 et d'un ou plusieurs autres monomères, où :
R⁶ est un groupe alkyle ou cycloalkyle contenant 1 à 30 atomes de carbone, -(CH₂)ₛR⁸, ou -(CH₂)ₜR⁵ ;
R⁸ est -C≡CH, -CH=CH₂ ou 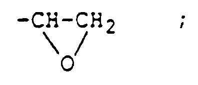 R⁵ est un groupe aryle ou aryle substitué ;
p est un nombre entier de 0 à 20 ;
q est un nombre entier de 1 à 10 ;
r est 0 ou 1 ;
s est 1, 2, 3 ou 4 ; et
t est 0, 1, 2, 3 ou 4 ;
à condition que si r est 0, p ne soit pas 0.

20. Le copolymère de la revendication 19, dans lequel ledit p est 0, ledit r est 1 et ledit q est de 1 à 10.

21. Le copolymère de la revendication 19, dans lequel ledit p est 1, ledit r est 1 et ledit q est 2.

22. Le copolymère tel que spécifié dans la revendication 19, dans lequel R¹ est un groupe :
*n*-alkyle ;
-(CH₂)ₛR³ où s est 1 et R³ est -C≡CH, -CH=CH₂, ou 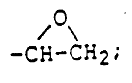 ou
-(CH₂)ₜR⁵ où t est 0 ou 1.

23. Le copolymère de la revendication 19, dans lequel ledit t est 0, ledit R⁵ est substitué par des groupes électro-attractifs ou porte des substituants dérivés de tels groupes.

24. Le copolymère de la revendication 22, dans lequel ledit p est 0, ledit r est 1 et ledit q est de 1 à 10.

25. Le copolymère de la revendication 22, dans lequel ledit p est 1, ledit r est 1 et ledit q est 2.

26. Le copolymère de la revendication 19, qui est un copolymère formé avec un monomère vinylique ou l'éthylène.

27. Le copolymère de la revendication 26, dans lequel ledit monomère vinylique est un monomère vinylique fluoré.

28. Le copolymère de la revendication 27, dans lequel ledit monomère vinylique fluoré est le fluorure de vinylidène, le tétrafluoréthylène, le fluorure de vinyle, le perfluoro(éther de méthyle et de vinyle), le chlorotrifluoréthylène, le perfluoro-1-heptène, l'hexafluoropropylène ou le perfluoro-2,2-diméthyl-1,3-dioxole.

29. Le copolymère de la revendication 28, dans lequel ledit monomère vinylique fluoré est le tétrafluoréthylène.

30. Le copolymère de la revendication 24, qui est un copolymère formé avec un monomère vinylique fluoré.

31. Le copolymère de la revendication 25, qui est un copolymère formé avec un monomère vinylique fluoré.

32. Le copolymère de la revendication 30, dans lequel ledit monomère vinylique fluoré est le fluorure de vinylidène, le tétrafluoréthylène, le fluorure de vinyle, le perfluoro(éther de méthyle et de vinyle), le chlorotrifluoréthylène, le perfluoro-1-heptène, l'hexafluoropropylène ou le perfluoro-2,2-diméthyl-1,3-dioxole.

33. Le copolymère de la revendication 31, dans lequel ledit monomère vinylique fluoré est le fluorure de vinylidène, le tétrafluoréthylène, le fluorure de vinyle, le perfluoro(éther de méthyle et de vinyle), le chlorotrifluoréthylène, le perfluoro-1-heptène, l'hexafluoropropylène ou le perfluoro-2,2-diméthyl-1,3-dioxole.

34. Le copolymère de la revendication 19, dans lequel lesdits motifs de la formule III 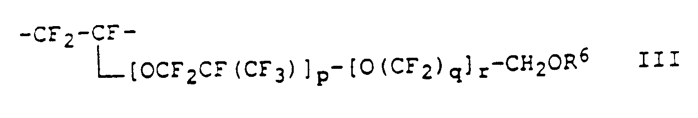 constituent environ 20 moles pour cent ou moins du polymère.

35. Le copolymère de la revendication 32, dans lequel lesdits motifs de la formule III 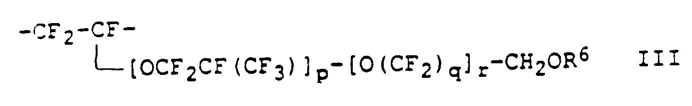 constituent environ 20 moles pour cent ou moins du polymère.

36. Un polymère produit par la polymérisation radicalaire de :
1) un composé de la formule IV
CF₂=CF-[OCF₂CF(CF₃)]ₚ-[O(CF₂)_{q}]ᵣ-CH₂OR⁷ IV
ou
2) un composé de formule IV et d'autres monomères vinyliques,
où
R⁷ est 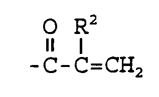 ou -(CH₂)ₛR⁴ ;
R² est l'hydrogène ou un groupe méthyle ;
R⁴ est un groupe styryle ;
p est un nombre entier de 0 à 20 ;
q est un nombre entier de 1 à 10 ;
r est 0 ou 1 ; et
s est 1, 2, 3 ou 4 ;
à condition que si r est 0, p ne soit pas 0.

37. Un polymère de la revendication 36, dans lequel ledit p est 0, ledit r est 1 et ledit q est de 1 à 10.

38. Un polymère de la revendication 36, dans lequel ledit p est 1, ledit r est 1 et ledit q est 2.

39. Un polymère de la revendication 36, dans lequel ledit s est 1.

40. Un polymère de la revendication 37, dans lequel ledit s est 1.

41. Un polymère de la revendication 38, dans lequel ledit s est 1.

42. Un polymère de la revendication 36, dans lequel ledit composé de la formule IV
CF₂=CF-[OCF₂CF(CF₃)]ₚ-[O(CF₂)_{q}]ᵣ-CH₂OR⁷ IV
constitue environ 20 moles pour cent ou moins du nombre total des motifs monomères du copolymère.

43. Le polymère de la revendication 36, qui est un copolymère formé avec l'un ou plusieurs de : esters acryliques, acrylamides, styrène, acétate de vinyle, propylène, tétrafluoréthylène, chlorotrifluoréthylène, fluorure de vinyle, fluorure de vinylidène, hexafluoropropylène ou perfluoro-2,2-diméthyl-1,3-dioxole.
